# EUROPEAN PATENT SPECIFICATION

(11) **EP 2 143 792 B1**
(45) Date of publication and mention of the grant of the patent: **08.01.2014**
(21) Application number: 08764303.7
(22) Date of filing: 09.05.2008
(51) Int. Cl.: C12N 15/11, C12N 15/113, A61K 31/7088, A61K 31/713

(54) **Single-stranded cyclic RNA, and method of production thereof**
Einstrangige zyklische RNS und Herstellungsverfahren dafür
ARN cyclique simple brin et procédé destine à produire celui-ci

(30) Priority: 09.05.2007 JP 2007125045
(43) Date of publication of application: 13.01.2010
(73) Proprietor: Riken, Wako-shi, Saitama 351-0198 (JP); Otsuka Pharmaceutical Co., Ltd., Tokyo 101-8535 (JP); Hayashi Kasei Co., Ltd., Osaka 530-0041 (JP)
(72) Inventor: ABE, Hiroshi, Wako-shi Saitama 351-0198 (JP); ITO, Yoshihiro, Wako-shi Saitama 351-0198 (JP); ABE, Naoko, Wako-shi Saitama 351-0198 (JP); TOYOBUKU, Hidekazu, Tokushima-shi Tokushima 771-0182 (JP)
(74) Representative: Woods, Geoffrey Corlett
(86) International application number: PCT/JP2008/058990
(87) International publication number: WO 2008/140126

(56) References cited:
- WO-A1-2005/014810
- WO-A2-00/63364
- WO-A2-03/070918
- WO-A2-2005/001063
- JP-A- 2005 517 423
- JP-A- 2005 517 427
- JP-A- 2005 517 430
- US-A1- 2004 138 166
- US-B1- 6 210 931
- BIAO DING ET AL: "Viroid: A Useful Model for Studying the Basic Principles of Infection and RNA Biology", MOLECULAR PLANT-MICROBE INTERACTIONS, vol. 20, no. 1, 1 January 2007 (2007-01-01), pages 7-20, XP55001077, ISSN: 0894-0282, DOI: 10.1094/MPMI-20-0007
- FLORES R ET AL: "Viroids: the minimal non-coding RNAs with autonomous replication", FEBS LETTERS, ELSEVIER, AMSTERDAM, NL, vol. 567, no. 1, 1 June 2004 (2004-06-01), pages 42-48, XP004511904, ISSN: 0014-5793, DOI: DOI:10.1016/J.FEBSLET.2004.03.118
- MA: "Design and synthesis of RNA miniduplexes via a synthetic linker approach. 2. Generation of covalently closed, double-stranded cyclic HIV-1 TAR RNA analogs with high Tat-binding affinity.", NUCLEIC ACIDS RESEARCH, vol. 21, no. 11, 1 January 1993 (1993-01-01), page 2585, XP55001092, ISSN: 0301-5610
- BOHJANEN P R ET AL: "A SMALL CIRCULAR TAR RNA DECOY SPECIFICALLY INHIBITS TAT-ACTIVATED HIV-1 TRANSCRIPTION", NUCLEIC ACIDS RESEARCH, OXFORD UNIVERSITY PRESS, SURREY, GB, vol. 24, no. 19, 1 October 1996 (1996-10-01), pages 3733-3738, XP000626571, ISSN: 0305-1048, DOI: DOI:10.1093/NAR/24.19.3733
- ERIE D A ET AL: "MELTING BEHAVIOR OF A COVALENTLY CLOSED, SINGLE-STRANDED, CIRCULAR DNA", BIOCHEMISTRY, AMERICAN CHEMICAL SOCIETY, vol. 28, no. 1, 1 January 1989 (1989-01-01), pages 268-273, XP002027684, ISSN: 0006-2960, DOI: DOI:10.1021/BI00427A037
- ASHLEY G W ET AL: "Chemical synthesis of oligodeoxynucleotide dumbbells", BIOCHEMISTRY, AMERICAN CHEMICAL SOCIETY, vol. 30, no. 11, 1 January 1991 (1991-01-01), pages 2927-2933, XP002975791, ISSN: 0006-2960, DOI: DOI:10.1021/BI00225A028
- YAMAKAWA H ET AL: "Properties of nicked and circular dumbbell RNA/DNA chimeric oligonucleotides containing antisense phosphodiester oligodeoxynucleotides", BIOORGANIC & MEDICINAL CHEMISTRY, PERGAMON, GB, vol. 6, no. 7, 1 July 1998 (1998-07-01), pages 1025-1032, XP027411625, ISSN: 0968-0896 [retrieved on 1998-07-01]
- ABE N. ET AL.: 'Dumbbell-Shaped Nanocircular RNAs for RNA Interference' J. AM. CHEM. SOC. vol. 129, no. 49, 12 December 2007, pages 15108 - 15109, XP008120979
- ABE N. ET AL.: 'Dumbbell-Shaped Nanocircular RNA o Mochiita RNA Kanshoho' CSJ: THE CHEMICAL SOCIETY OF JAPAN TAIKAI KOEN YOKOSHU vol. 88TH, 12 March 2008, page 825 + ABSTR. NO. 3 A4-49, XP008121161
- HARADA M. ET AL.: 'Kagaku Shushoku shita Dumbbell-Shaped Nanocircular RNA o Mochiita RNA Kanshoho no Kento' CSJ: THE CHEMICAL SOCIETY OF JAPAN TAIKAI KOEN YOKOSHU vol. 88TH, 12 March 2008, page 825 + ABSTR. NO. 3 A4-50, XP008121162

## Description

### Technical Field

The present invention relates to a single-chain circular RNA, a method of producing the RNA, and a pharmaceutical composition comprising the RNA.

### Background Art

Conventional RNA interference methods are generally classified into two groups: a method using chemically synthesized double-stranded RNAs and a method using plasmid vectors. The RNA interference method using plasmid vectors is widely used in the field of biotechnology, mainly in basic biology experiments. However, when the RNA interference method is applied to developing pharmaceutical preparations, the method using plasmid vectors has a problem of safety to a human body. Hence, it is likely to be more preferable to use the chemically synthesized double-stranded RNAs.

However, there is the problem that chemically synthesized double-stranded RNAs are unstable *in vivo,* namely, susceptible to degradation with enzymes (nucleases) in cells. To solve this problem, RNA strands with non-natural nucleic acids have been developed in order to enhance stability of double-stranded RNAs in cells; however, there exists another problem that its biological activity reduces while the stability is improved. Additionally, toxicity caused by non-natural nucleic acids is unknown. Therefore, it remains difficult to achieve the application to pharmaceutical preparations.

The forms of a double-stranded RNA usable for the RNA interference method include a double-stranded RNA having blunt ends or protruding ends, an RNA having a hairpin structure with a loop at either end of the double-stranded RNA (JP2003-502012A), a circular nucleic acid which contains approximately 19 base pairs, two loops, and optionally chemically modified polynucleotides (JP2006-271387A), and the like. However, this circular nucleic acid is not subjected to testing for RNA interference effect.

In addition, an RNA-DNA chimeric dumbbell-shaped nucleic acid is disclosed (JP11-137260A(1999)). This nucleic acid is not for use in RNA interference. The RNA portion of the dumbbell-shaped nucleic acid is cleaved by an enzyme in cells, and the resulting antisense DNA portion binds to a mRNA in cells to inhibit it. JP11-137260A(1999) discloses that a nucleic acid possesses a high resistance to nucleic acid degrading enzymes in cells and remains stable in cells until ribonuclease H acts, owning to its dumbbell-shaped structure. It further discloses that, because a single-chain oligonucleotide containing the antisense DNA portion can be released into the cytoplasm of cells only after cleaving the complementary RNA portion with the effect of ribonuclease H, the antisense effect per dose is believed to be enhanced.

Moreover, regarding the method of synthesizing a circular nucleic acid having a dumbbell-shaped structure, for example, it is disclosed that linear oligonucleotides are synthesized, a stem region and a hairpin loop region are formed to obtain a nick dumbbell-shaped oligonucleotide, wherein one site of the target circular dumbbell-shaped oligonucleotide (the opposite end of the above hairpin loop region) is unbound, and the 5' end is ligated with a ligase to prepare a circular dumbbell-shaped circular nucleic acid (JP11-137260(1999)).

It is an object of the present invention to provide a single-chain circular RNA and a method of producing the same.

### Disclosure of the Invention

The present inventors have now found that, surprisingly, a single-chain circular RNA can be obtained with a high yield by separately synthesizing a sense strand and an antisense strand, both comprising a nucleotide sequence with unpaired nucleotides at each end, and allowing ligase to act on the nucleotides at both ends simultaneously, and that the obtained single-chain circular RNA exerts a sustained or slow-releasing RNA interference effect. Based on these scientific findings, the present inventors have accomplished the present invention.

More specifically, the present invention includes the following
(1) A single-chain circular RNA having a sustained or slow-releasing RNA interference effect, characterized in that the single-chain circular RNA comprises a sense strand sequence, an antisense strand sequence complementary to the sense strand sequence, identical or different two loop sequences between the sense strand and the antisense strand, connecting both strands, wherein the sense strand and the antisense strand are paired to form a stem, and wherein each of the two loop sequences has 6 to 9 nucleotides in length.
(2) The single-chain circular RNA according to the above (1), wherein the expression of a target RNA is 0.4 or less 24 hours after introduction of the single-chain circular RNA into eukaryotic cells, compared to control cells whose expression level of the target RNA is set to 1.
(3) The single-chain circular RNA according to the above (1) or (2), wherein 70% or more thereof is retained after 8 hours in human serum.
(4) A method of producing the single-chain circular RNA according to any one of the above (1) to (3), comprising synthesizing a sense strand and an antisense strand, both comprising a nucleotide sequence with unpaired nucleotides at the 5' end and 3' end, and simultaneously ligating the nucleotide at the 5' end of the nucleotide sequence with unpaired nucleotides in the sense strand, with the nucleotide at the 3' end of the nucleotide sequence with unpaired nucleotides in the antisense strand, and vice versa, using a ligase,
   wherein the nucleotide sequence with unpaired nucleotides at the 5' end of the sense strand and the nucleotide sequence with unpaired nucleotides at the 3' end of the antisense strand are bound to each other to form a loop, the nucleotide sequence with unpaired nucleotides at the 3' end of the sense strand and the nucleotide sequence with unpaired nucleotides at the 5' end of the antisense strand are bound to each other to form a loop, and the sense strand and the antisense strand are paired to form a stem.
(5) The method according to the above (4), further comprising phosphorylating each 5' end of the nucleotide sequence with unpaired nucleotides in the sense strand and the nucleotide sequence with unpaired nucleotides in the antisense strand.
(6) The method according to the above (4) or (5), wherein the loop sequences are identical to or different from each other.
(7) The method according to any one of the above (4) to (6), wherein the stem length is 19 to 31 nucleotides.
(8) A method of suppressing expression of a gene encoding a protein *in vitro,* comprising introducing the single-chain circular RNA according to any one of the above (1) to (3) to human-derived cells, and impairing a target RNA by the single-chain circular RNA to inhibit translation of the target RNA into the protein in a sustained manner.
(9) A method of suppressing expression of a gene encoding a protein, comprising introducing in vitro the single-chain circular RNA according to any one of the above (1) to (3) to cells of a non-human animal or plant, and impairing a target RNA by the single-chain circular RNA to inhibit translation of the target RNA into the protein in a sustained manner.
(10) A pharmaceutical composition comprising the single-chain circular RNA according to any one of the above (1) to (3) as an active ingredient.

As used herein, the term "single-chain circular RNA" may be referred to as dumbbell-shaped RNA. A dumbbell-shaped RNA refers to a single-chain circular RNA, wherein a sense strand and an antisense strand are complementarily paired to form a stem, loops are formed at both sides of the stem by a nucleotide sequence with unpaired nucleotides, and the entire shape of the single-chain circular RNA is in a dumbbell.

According to the present invention, the dumbbell-shaped synthetic RNA, which is excellent in stability, sustainability and slow-releasing property, can be efficiently produced.

In particular, the dumbbell-shaped RNA for use in the RNA interference method can be produced by cyclizing two RNA strands. Because both ends are closed in a loop shape, it has no RNA end and is therefore not likely to serve as a substrate for enzymes, exonuclease (RNase) and the like, except for specific enzymes such as Dicer in cells. Thus, it is less susceptible to enzymatic degradation and has significantly increased stability in the cell. As a result, there is no necessity to use non-natural nucleic acids to enhance stability.

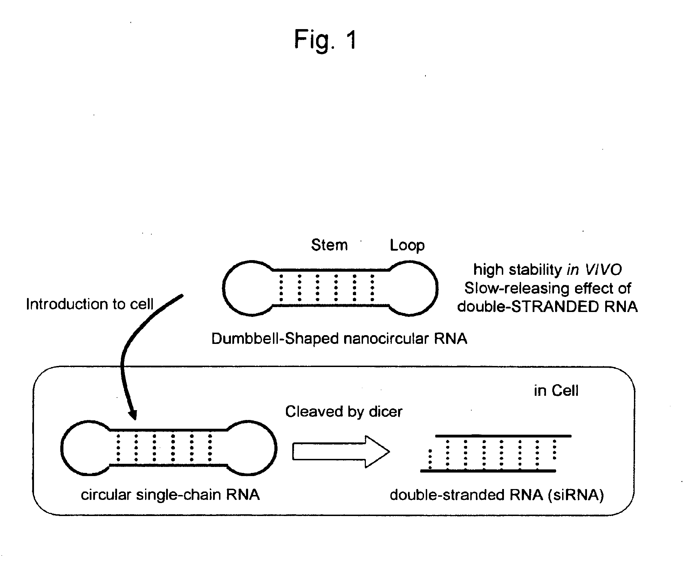

Moreover, the dumbbell-shaped RNA is specifically recognized by *in vivo* enzymes such as Dicer in cells, and the loop regions at both sides are cleaved to form a naturally occurring type double-stranded RNA (Figure 1). As a result, it can have activity equivalent to that of a double-stranded RNA, and exerts a more sustainable or slow-releasing effect than RNA interference effect by conventional double-stranded RNAs.

The dumbbell-shaped RNA of the present invention is also more stable than conventional double-stranded RNAs in human serum.

### Brief Description of the Drawings

Figure 1 is a schematic view of the dumbbell-shaped RNA of the present invention.
Figure 2 shows the structure of RNAs and an electrophoresis image of each RNA.
Figure 3 shows the structure of siRNA and dumbbell-shaped RNAs.
Figure 4 shows electrophoresis images of dumbbell-shaped RNAs and liner double-stranded RNAs cleaved by Dicer.
Figure 5 shows an interference effect of each dumbbell-shaped RNA 24 hours after transfection.
Figure 6 shows a sustainable RNA interference effect of the dumbbell-shaped RNA.

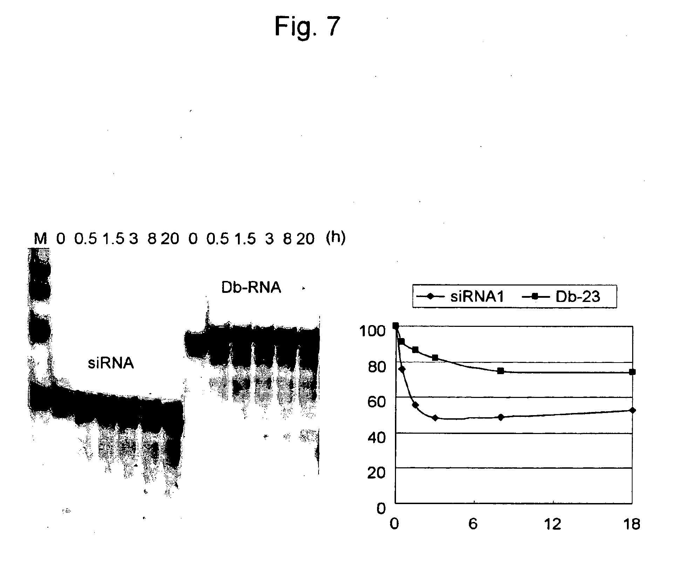

Figure 7 shows stability of the dumbbell-shaped RNA in human serum.

### Best Mode for Carrying Out the Invention

The single-chain circular RNA of the present invention includes a sense strand sequence homologuous to the nucleotide sequence of a target RNA or part thereof, an antisense strand sequence which is complementary to the sense strand sequence and is capable of pairing with it, and loop sequences which cannot form pairing between the strands.

The sense strand and the antisense strand are paired to form a stem. The length of the stem, not specifically limited, can be determined depending on the type, structure or the like of a target RNA. For example, the stem is composed of 19 to 31 base pairs, preferably 21 to 25 base pairs, more preferably 22 to 24 base pairs, and even more preferably 23 base pairs.

The nucleotide sequences with unpaired nucleotides are present at the 5' end and 3' end of both the sense strand and the antisense strand. The nucleotide at the 5' end of the nucleotide sequence with unpaired nucleotides in the sense strand and the nucleotide at the 3' end of the nucleotide sequence with unpaired nucleotides in the antisense strand are ligated together to form a loop. The nucleotide at the 3' end of the nucleotide sequence with unpaired nucleotides in the sense strand and the nucleotide at the 5' end of the nucleotide sequence with unpaired nucleotides in the antisense strand are ligated together to form a loop. The loop length is 6 to 9 bases. Therefore, the entire single-chain circular RNA is preferably composed of 42 to 80 bases.

Where the nucleotide sequence with unpaired nucleotides at the 5' end of the sense strand is represented as A, the nucleotide sequence with unpaired nucleotides at the 3' end of the antisense strand is represented as B, the nucleotide sequence with unpaired nucleotides at the 3' end of the sense strand is represented as C, and the nucleotide sequence with unpaired nucleotides at the 5' end of the antisense strand is represented as D, for example, if the loop length is 20 bases, then A is 1 to 19 bases in length, B is 19 to 1 bases in length, C is 19 to 1 bases in length, and D is 1 to 19 bases in length. Therefore, the nucleotide at the 5' end of A and the nucleotide at the 3' end of B are ligated together to form a 20-base loop, and the nucleotide at the 3' end of C and the nucleotide at the 5' end of D are ligated together to form a 20-base loop. Although loops of 20 bases are not part of the present invention, the same principle applies to loops of 6 to 9 bases.

It is preferable that the sequences, which cannot form pairing with each other, vary in length between A and B, and between C and D, for example, by 1 base, 2 bases or 3 bases or, alternatively, that the sequences are of the same length. Therefore, for example, where the loop length is 8 bases, it is preferable that A is 3 to 5 bases in length, B is 5 to 3 bases in length, C is 5 to 3 bases in length, and D is 3 to 5 bases in length. Where the loop length is 9 bases, it is preferable that A is 3 to 6 bases in length, B is 6 to 3 bases in length, C is 6 to 3 bases in length, and D is 3 to 6 bases in length.

In addition, the nucleotide sequences of the loop formed by A and B and the loop formed by C and D may be identical or different.

The single-chain circular RNA of the present invention can be used for the RNA interference method.

RNA interference is also known as RNAi, and is a phenomenon in which a small RNA molecule having a sequence complementary to a target RNA binds to the target RNA, thereby degrading the target RNA or suppressing the translation of the target RNA.

The antisense strand of the dumbbell-shaped RNA has a sequence complementary to a target RNA (for example, mRNA or the precursor RNA thereof). Moreover, the nucleotide sequences with unpaired nucleotides include, but not limited to, UUCAAGAGA and UGUGCUGUC (M. Miyagishi et al., Oligonucleotides 2003, Vol. 13: pp. 1-7).

Furthermore, the loop in the dumbbell-shaped RNA may be chemically modified. For example, *in vivo* stability of the dumbbell-shaped RNA can be enhanced by modifying with polyethylene glycol whose molecular weight is approximately 2000 to 5000. The loop of the dumbbell-shaped RNA is cleaved by an enzyme like Dicer in cells to be removed. Therefore, it is believed that polyethylene glycol has little effect when the stem exerts its RNA interference effect as an siRNA or miRNA.

With the dumbbell-shaped RNA of the present invention, the expression of a target RNA in cells, in which the dumbbell-shaped RNA has been introduced, is preferably 0.4 or less 24 hours after introduction of the dumbbell-shaped RNA into the cells, compared to control cells (without the dumbbell-shaped RNA being introduced) whose expression level of the target RNA is set to 1.

According to the present invention, the cells are eukaryotic cells, preferably animal cells and plant cells.

The expression of a target RNA can be confirmed by, for example, transforming cells with a reporter gene (e.g., luciferase, β-galactosidase, β-glucuronidase, or green fluorescent protein (GFP) gene), and measuring the coloring or fluorescence of the reporter gene-derived protein to examine the level of inhibition of a target RNA expression by the dumbbell-shaped RNA, wherein mRNA of the reporter gene may be used as the target RNA.

Moreover, the dumbbell-shaped RNA of the present invention is characterized in that 70% or more thereof are retained without being degraded after 8 hours in human serum. For example, this can be confirmed by incubating the dumbbell-shaped RNA in human serum and measuring the molecular weight by using electrophoresis or the like to test whether it is degraded over time.

The method of producing the dumbbell-shaped single-chain circular RNA of the present invention includes synthesizing a sense strand and an antisense strand, both comprising a nucleotide sequence with unpaired nucleotides at the 5' end and 3' end, and simultaneously ligating the nucleotide at the 5' end of the nucleotide sequence with unpaired nucleotides in the sense strand, with the nucleotide at the 3' end of the nucleotide sequence with unpaired nucleotides in the antisense strand, and vice versa, using a ligase.

The sense strand and the antisense strand can be designed to suppress the function of a target gene, based on the nucleotide sequence of the target gene. The designs can be confirmed by producing multiple sense and antisense strands and testing for each suppression efficiency. For example, designing using an algorithm for siRNA design or the like can be applied (References: J. A. Jaeger et al., Methods in Enzymology (1989) 183: 281-306; D. H. Mathews et al., J. Mol. Biol. (1999) 288: 911-940). When designing, it is preferable that the strands do not suppress the expression of genes other than a target gene, the genes having sequences similar to the target gene (which is known as the off target effect). The lengths of the sense and the antisense strands are preferably designed in the range of, for example, 19 to 31 bases, preferably 21 to 25 bases, more preferably 22 to 24 bases, and even more preferably 23 bases.

The target gene includes, but not limited to, abl/bcr gene for leukemia, VEGF gene for age-related macular degeneration, and HCV gene for hepatitis.

A sequence which subsequently forms a loop, for example the above sequence UUCAAGAGA, is divided into two fragments at an arbitrary position to form a nucleotide sequence with unpaired nucleotides (wherein the sequence is 9 bases, and when divided into two fragments, as described above, the difference in length is preferably in the range of 1 to 3 bases). A sequence is designed, so that one fragment is ligated to the 3' end of the antisense strand, and the other is ligated to the 5' end of the sense strand. By the same method, another sequence is designed so that one fragment is ligated to the 3' end of the sense strand, the other is ligated to the 5' end of the antisense strand. Single-chain nucleic acids having these two designed sequences are separately synthesized. In doing this, it is preferable to perform the 5' end phosphorylation by using a chemical phosphorylation reagent. Moreover, the method of the invention requires the design of a sequence to form a loop, whose length is 6 to 9 bases.

There are various methods for synthesizing nucleic acids such as *in vitro* transcription synthesis method, methods using plasmids or virual vectors, and methods using PCR cassettes. Although a method of synthesizing nucleic acids is not specifically limited, a chemical synthesis method is preferred in terms of high purity, ability to produce in large quantities, safety for use *in vivo,* ability of chemical modification, and the like. Examples of chemical synthesis method include, but not limited to, H-phosphonate method and phosphoroamidite method. For this purpose, commercially available automatic nucleic acid synthesizers may be used.

The ends of the nucleotide sequences with unpaired nucleotides at both ends of the sense strand and the antisense strand are ligated with a ligase (for example, T4 RNA ligase or T4 DNA ligase) to form two loops simultaneously. The reaction conditions include, for example, incubating in a buffer containing polyethylene glycol (PEG), BSA and the like for 20 hours at a low temperature. The synthesized dumbbell-shaped single-chain circular RNA can be collected and purified by ordinary methods (for example, high-performance liquid chromatography and PAGE method).

Moreover, the single-chain circular RNA may be chemically modified with polyethylene glycol (PEG) or the like, wherein the chemical modification is preferably performed at the loop region. For binding, both ends of PEG are modified to introduce a functional group reactive with the amino groups in bases, such as a formyl group or an N-hydroxysuccinimide ester group.

The RNA interference method using the dumbbell-shaped RNA produced by the above method is described hereinafter.

According to the present invention, the single-chain circular RNA of the present invention can be introduced in cells in vitro and impair a target RNA to inhibit the translation of the target RNA into a protein in a sustained manner, wherein human cells or non-human animal or plant cells can be used as the cells.

When the RNA interference method is performed *in vitro,* the dumbbell-shaped RNA is introduced into cells by, for example, electroporation method, microinjection method, lipofection method, or calcium phosphate transfection.

The dumbbell-shaped RNA introduced into cells is cleaved by Dicer in the cells to generate a double-stranded RNA (siRNA) which has an RNA interference effect (Figure 1). The ends of the siRNA can be either blunt ends or protruding ends. The siRNA turns into a single chain to form an RNA-nuclease complex (RNA induced silencing complex (RISC)), which recognizes a target mRNA having a sequence complementary to the siRNA, and degrades the target mRNA, thereby suppressing the expression of the corresponding target gene.

The single-chain circular RNA of the present invention can be used in any of plant cells, and animal (for example, humans, pets, mammals including domestic animals) cells. Wide applications in the fields of medicine and agriculture will be expected. For example, the single-chain circular RNA of the present invention can be used for various purposes including elucidation of the function of a specific gene or protein in plants or animals, or at plant or animal cellular level, by using for example knockout methods.

In addition, the present invention includes a pharmaceutical composition comprising the single-chain circular RNA as an active ingredient.

The amount of the single-chain circular RNA formulated in the pharmaceutical composition may be adjusted in accordance with the kind and purpose of the composition. For example, the amount of the RNA includes, but not limited to, 1 wt%, 3 wt%, 5 wt%, 10 wt%, 20 wt%, 30 wt%, 40 wt%, 50 wt%, 60 wt%, 70 wt%, 80 wt%, 90 wt% or 100 wt% relative to the total amount of the composition.

Examples of the pharmaceutical composition of the present invention include liquid preparations (such as solution, suspension, emulsion), solid preparations (such as freeze-dried preparation capable of being reconstituted before use), liposome (preferably, cationic liposome)-encapsulated preparations. In addition, preferred administration route is a parenteral administration, which includes, for example, local administration applying the preparation directly at an affected site, pulmonary administration, transmucosal administration such as nasal administration, and intravenous administration.

The pharmaceutical composition of the present invention may include excipients (saline, sterilized water, Ringer's solution and the like), buffering agents, tonicity agents, stabilizing agents, and the like, depending on formulations or dosage forms.

Moreover, the dosage of the pharmaceutical composition of the present invention may vary depending on sex, weight, age, severity, symptoms, or the like, of a patient.

The pharmaceutical composition of the present invention is applicable to, for example, treatment of diseases such as cancers (e.g., suppression of functions of genes or proteins which are specifically expressed in cancer cells).

The present invention will hereinafter be described further specifically by the following examples. However, it should be understood that the scope of the present invention is not limited to the specific examples.

### Examples

### Example 1: Preparation of a dumbbell-shaped RNA

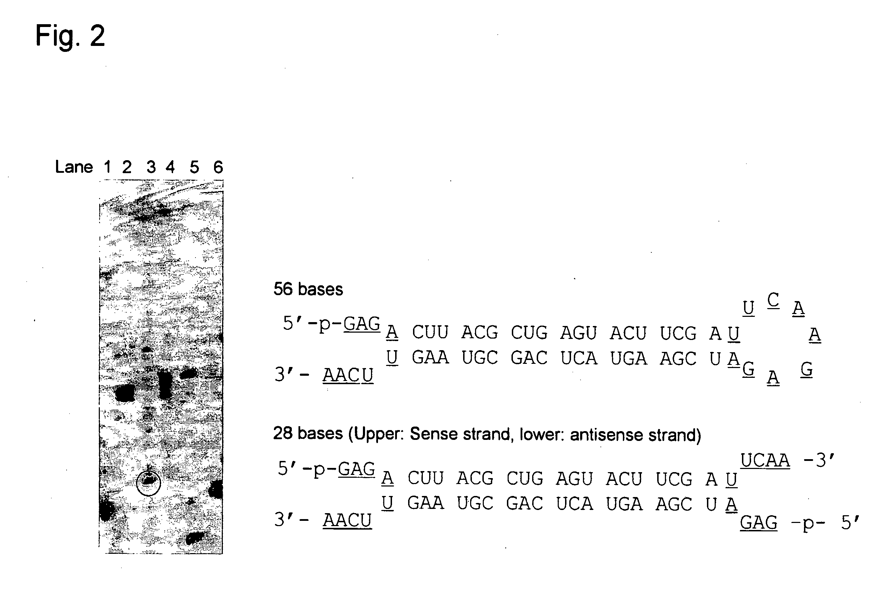

5'-phosphorylated RNAs serving as raw materials for dumbbell-shaped RNAs were all synthesized on DNA synthesizer (GeneWorld H8-SE) in accordance with the phosphoroamidite method. Protected TBDMS (Proligo Corp.) was used for RNA amidites, and Chemical Phosphorylation Reagent (Glen Research Corp.) was used for 5'-phosphorylation. Deprotection was performed by the ordinary method, followed by PAGE-purification. The sequences of the synthesized RNA are shown in SEQ ID NO: 1 (sense strand, 28 mer), SEQ ID NO: 2 (antisense strand, 28 mer), SEQ ID NO: 3 (56 mer), and in Figure 2. Moreover, in the RNA sequences shown in Figure 2, the underlined sequences are sequences which form the loop region of a dumbbell-shaped RNA.

Subsequently, enzymatic reaction was performed in the mixture of 2 µM RNA double strand, 2.0 units/µl T4 RNA ligase, 0.006% BSA, 25% PEG6000, 50 mM Tris-HCl (pH 7.5), 10 mM MgCl₂, 10 mM DTT and 1 mM ATP in a total volume of 25 µl.

More specifically, first, 5'-phosphorylated double-stranded RNA was dissolved to 12.5 µl of a buffer (2 x buffer) containing 100 mM Tris-HCl (pH 7.5), 20 mM MgCl₂, 20 mM DTT and 2 mM ATP, at a concentration of 4 µM. The solution was heated at 65°C for 5 minutes, and then slowly cooled to room temperature. Subsequently, BSA solution, PEG6000 solution and T4 RNA ligase (Takara Bio Inc.) were added to form the above composition and reaction volume. The solution was then incubated at 11°C for 20 hours. RNA was collected by ethanol precipitation and analyzed by PAGE.

The samples at each lane of PAGE in Figure 2 are as follows.
Lane 1: mixture of the 28-base sense strand and the 28-base antisense strand (marker)
Lane 2: 57-base RNA (marker)
Lane 3: dumbbell-shaped RNA formed from the 28-base sense strand and the 28-base antisense strand
Lane 4: dumbbell-shaped RNA formed from 56 bases (single-chain)
Lane 5: 28-base sense strand treated with RNA ligase (reference)
Lane 6: 28-base antisense strand treated with RNA ligase (reference)

The dumbbell-shaped RNA at Lane 4 was produced by synthesizing a single chain having 56 bases (SEQ ID NO: 3), forming a stem region and hairpin loop region, and ligating the first nucleotide and last nucleotide with T4 ligase.

The band surrounded by a circle on Lane 3 represents the dumbbell-shaped RNA produced by the method of the present invention, and the yield was approximately 80%. The dumbbell-shaped RNA at Lane 4 had a yield of approximately 5% or less.

### Example 2: Examination of the stem length of the dumbbell-shaped RNA

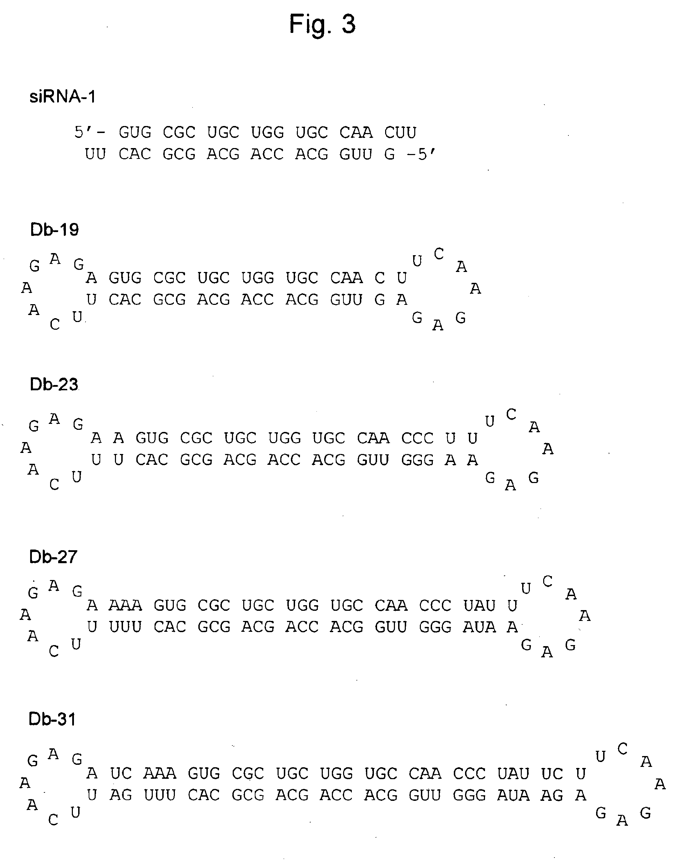

RNAs represented by SEQ ID NOS: 4 through 13 were synthesized by the above DNA synthesizer. SEQ ID NOS: 4 and 5 form a double-stranded RNA which forms 18 base pairs (siRNA-1), SEQ ID NOS: 6 and 7 form a dumbbell-shaped RNA whose stem length is 19 bases (Db-19), SEQ ID NOS: 8 and 9 form a dumbbell-shaped RNA whose stem length is 23 bases (Db-23), SEQ ID NOS: 10 and 11 form a dumbbell-shaped RNA whose stem length is 27 bases (Db-27), and SEQ ID NOS: 12 and 13 form a dumbbell-shaped RNA whose stem length is 31 bases (Db-31) (Figure 3).

An enzymatic reaction was performed in the composition of 2 µM RNA double strand, 1.0 units/µl T4 RNA ligase, 0.006% BSA, 25% PEG6000, 50 mM Tris-HCl (pH 7.5), 10 mM MgCl₂, 10 mM DTT and 1 mM ATP in a reaction volume ranging from 1.5 to 2.5 ml.

More specifically, 5'-phosphorylated double-stranded RNA was dissolved in a buffer (2 x buffer, half of the amount of final reaction solution) containing 100 mM Tris-HCl (pH 7.5), 20 mM MgCl₂, 20 mM DTT and 2 mM ATP, at a concentration of 4 µM. The solution was heated at 65°C for 5 minutes and then slowly cooled to room temperature. Subsequently, BSA solution, PEG6000 solution and T4 RNA ligase (Takara Bio Inc.) were added to form the above composition and reaction volume. The solution was then incubated at 11°C for 20 hours.

RNA was collected by alcohol precipitation from the reaction solution, and the cyclized products were isolated by using PAGE. Bands containing the targets were visualized by the UV shadowing method, cut out, crushed into small pieces, and extracted 3 times with 4 ml of an elution buffer (0.2 M NaCl, 1 mM EDTA (pH 8.0)). The extract was desalted with a Sep-Pak cartridge (eluted with 6 ml of 50% acetonitrile in water), condensed with a centrifugal evaporator, and further subjected to alcohol precipitation in the presence of ammonium acetate. The targets were quantified by measuring UV spectra.

Two units of ColdShock-DICER (Takara Bio Inc.) was added to 2 µg of each of the above dumbbell-shaped RNAs in a buffer containing 20 mM Tris-HCl (pH 8.5), 150 mM NaCl and 2.5 mM MgCl₂ (reaction volume: 20 µl), and the resulting solution was then incubated at 37°C. After 1, 6 and 18 hours, 3 µl of the reaction solution was collected as a sample. Each sample was mixed with 2 µl of 120 mM EDTA solution to terminate the enzymatic reaction. These were then analyzed by native PAGE (10% PAGE, 1 x TBE) (after being stained with 1 x SYBR Green I, the samples were imaged and quantified on a BioRad Molecular Imager FX).

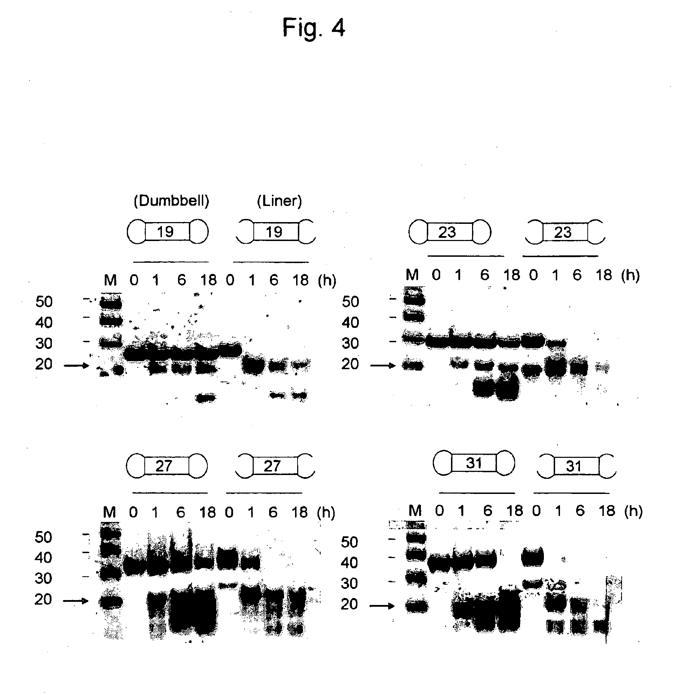

Figure 4 shows PAGE of each dumbbell-shaped RNA. As a control, a cleavage reaction using the double-stranded RNA prior to a dumbbelling reaction as a substrate was examined by the same method. As a result, the cleavage reaction of Db-19 proceeded approximately 5% after 1 hour, and the production of double-stranded RNAs of 20 bases in length was confirmed. The cleavage fragments of Db-19 in the range of 20 bases maintained almost the same level of concentration after 6 and 18 hours. In contrast, almost 100% of the control including 19-base pair linear chain (Liner) was cleaved after 1 hour, and double-stranded RNAs of 20 bases in length were observed. Subsequently, double-stranded RNAs of the product were degraded and disappeared over time. With Db-23, approximately 8% was cleaved after 1 hour, and double-stranded RNAs of 20 bases in length were produced. It was confirmed that as the stem length increases, the cleavage speed after 1 hour increases to 10% and 20% in Db-27 and Db-31, respectively. After 18 hours, approximately 75% of Db-19 remains intact whereas Db-31 completely disappeared. From these results, it was revealed that the dumbbell-shaped RNAs with shorter stem length are more stable against an enzyme.

### Example 3: RNA interference effect of the dumbbell-shaped RNA

RNA interference effect of the above dumbbell-shaped RNAs (Db-19, Db-23, Db-27 and Db-31) was evaluated by an inhibition experiment of the expression of firefly luciferase reporter gene pGL3.

NIH 3T3 cells (Riken Cell Bank) were cultured in DMEM (GIBCO) medium containing 10% FCS at 37°C in 5% CO₂, and a 100 µl aliquot of the culture was inoculated to each well of a 96-well plate at a concentration of 1.6 x 10⁴ cells/well. The sample was further cultured at 37°C in 5% CO₂ for 39 hours to give approximately 70% confluence. The cells were then cotransfected with 2 kinds of plasmid vectors (pGL3-Control and pRL-TK (for internal standard), from Promega Corp.) and each kind of RNAs, using the transfection reagent GeneSilencer (Genlantis Inc.) in accordance with the protocol attached to the transfection reagent. The concentration conditions at the time of transfection are as follows.

0.2 µg pGL3-Control/ 0.02 µg pRL-TK/ 25 nM RNA (Final volume 100 µl)

| | |
|---|---|
| GeneSilencer | 1 µl |
| DMEM medium | 25 µl |
| siRNA diluted solution | 2.5 µl |
| DMEM medium | 15 µl |
| RNA (5 pmol/µl) | 0.5 µl |
| pGL3-Control (1 µg/µl) | 0.2 µl |
| pRL-TK (0.1 µg/µl) | 0.2 µl |
| | 44.4 µl |

After transfection, the culture was incubated at 37°C in 5% CO₂ for 4 hours. One hundred µl of DMEM medium containing 20% serum was then added to each well. After further incubation at 37°C for 20 hours, the cells were solubilized to quantify the expression level of luciferase using Dual-luciferase Reporter Assay System (Promega Corp.) in accordance with the protocol attached (Conditions: the amount of reagent 30 µl, delay time 2 seconds, reading time 10 seconds. Equipment: Wallac ARVO SX 1420 Multilabel Counter).

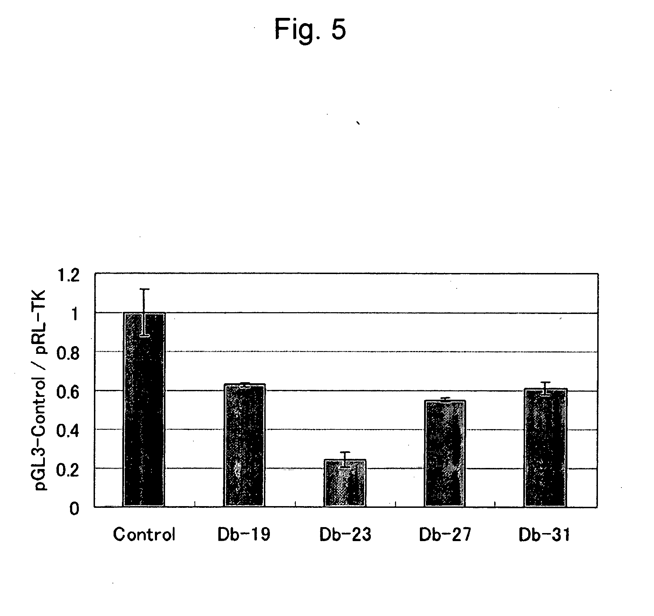

For comparison, a control (buffer only) was evaluated by the same method. The fluorescence intensity of firefly luciferase was corrected for the fluorescence intensity of renilla luciferase as an internal standard. The results are shown in Figure 5. After 24 hours, among the dumbbell-shaped RNAs, Db-23 showed the highest activity, and exhibited an inhibition effect as low as 0.24. From these results, the most preferred double-strand length was set to 23 bases.

### Example 4: Sustainability of RNA interference effect

Sustainability ofRNA interference effect of Db-23 and siRNA-1 was compared.

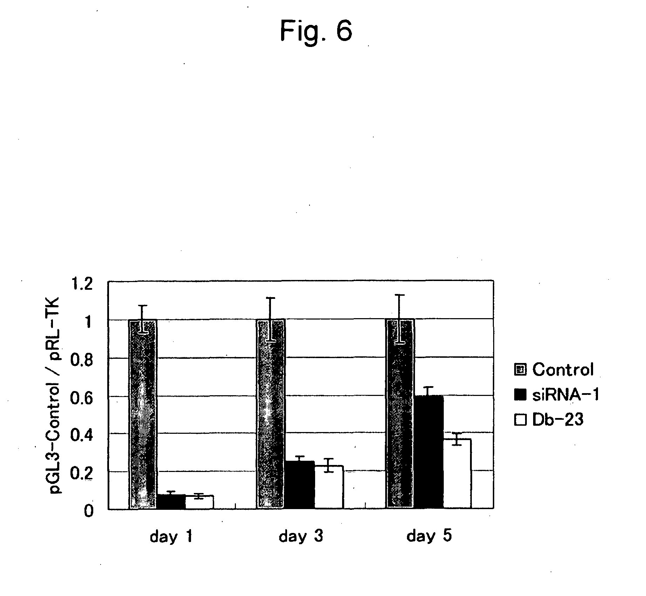

NIH 3T3 cells were cultured in Dulbeco's Modified Eagles Medium (DMEM, Gibco) supplemented with 10% fetal calf serum (FCS, Invitro/Gibco) in a 5% CO₂-humidified chamber. 40 hours before transfection at about 70% confluent, cells were seeded in 96-well plates at a density of 1.6 x 10⁴ cells per well (100 µl). Co-transfection of reporter plasmids and RNA was carried out with GeneSilencer (Gene Therapy systems, Inc.) as described by the manufacturer for adherent cell lines. Per well, 16 ng/µl or 1.6 ng/µl pGL3-Control (Promega Corp.), 1.6 ng/µl pRL-TK (Promega Corp.) and 25 nM RNA formulated with the transfection reagent were applied (100 µl). After 4 h incubation, 100 µl of 20% FCS in DMEM was added. For a prolonged incubation longer than 3 days, the medium was replaced as needed. Luciferase expression was monitored after 24 hours, 72 hours, and 120 hours with Dual-Luciferase Reporter Assay System (Promega Corp.) according to the instructions provided on Wallac ARVO SX 1420 Multilabel Counter (Perkin-Elmer, Inc.) (Fig. 6). A sample without RNA was used as a control.

Db-23 and siRNA-1 both showed almost equal level of luciferase activity after 24 hours. However, after 120 hours, Db-23 showed a higher gene expression suppression effect. From these results, both the slow-releasing effect and long-term activation effect of the dumbbell-shaped RNA were shown.

### Example 5: Stability of the dumbbell-shaped RNA in human serum

Stability of Db-23 and siRNA in human serum was compared. 4 µl of pre-annealed dsRNA (siRNA-1, 20 µM) or dumbbell RNA (Db-23, 20 µM) in annealing buffer (100 mM potassium acetate, 30 mM HEPES-KOH (pH7.4), 2 mM magnesium acetate) was mixed with 32 µl of PBS, 4 µl of Normal Human Serum (Chemicon International, Inc.), incubated at 37 °C. Aliquot (5 µl) was taken after 0.5, 1, 1.5, 3, 8 and 20 hours. The reaction was analyzed by 15% native PAGE, stained with SYBR Green I and visualized with Molecularlmager FX (BioRad Laboratories, Inc.).

As a result, up to 50% of siRNA was degraded after 3 hours, whereas 80% or more of Db-23 still remained after 3 hours, and 70% or more even after 8 hours. Therefore, high stability of Db-23 *in vivo* could be expected.

### Industrial Applicability

The present invention can be used as a nucleic acid molecule applicable to living organisms and can produce the molecule with a high yield.

## Claims

1. A single-chain circular RNA having a sustained or slow-releasing RNA interference effect, **characterized in that** the single-chain circular RNA comprises a sense strand sequence, an antisense strand sequence complementary to the sense strand sequence, identical or different two loop sequences between the sense strand and the antisense strand, connecting both strands, wherein the sense strand and the antisense strand are paired to form a stem, and wherein each of the two loop sequences has 6 to 9 nucleotides in length.

2. The single-chain circular RNA according to claim 1, wherein the expression of a target RNA is 0.4 or less 24 hours after introduction of the single-chain circular RNA into eukaryotic cells, compared to control cells whose expression level of the target RNA is set to 1.

3. The single-chain circular RNA according to claim 1 or 2, wherein 70% or more thereof is retained after 8 hours in human serum.

4. A method of producing the single-chain circular RNA according to any one of claims 1 to 3, comprising synthesizing a sense strand and an antisense strand, both comprising a nucleotide sequence with unpaired nucleotides at the 5' end and 3' end, and simultaneously ligating the nucleotide at the 5' end of the nucleotide sequence with unpaired nucleotides in the sense strand, with the nucleotide at the 3' end of the nucleotide sequence with unpaired nucleotides in the antisense strand, and vice versa, using a ligase,
wherein the nucleotide sequence with unpaired nucleotides at the 5' end of the sense strand and the nucleotide sequence with unpaired nucleotides at the 3' end of the antisense strand are bound to each other to form a loop, the nucleotide sequence with unpaired nucleotides at the 3' end of the sense strand and the nucleotide sequence with unpaired nucleotides at the 5' end of the antisense strand are bound to each other to form a loop, and the sense strand and the antisense strand are paired to form a stem.

5. The method according to claim 4, further comprising phosphorylating each 5' end of the nucleotide sequence with unpaired nucleotides in the sense strand and the nucleotide sequence with unpaired nucleotides in the antisense strand.

6. The method according to claim 4 or 5, wherein the loop sequences are identical to or different from each other.

7. The method according to any one of claims 4 to 6, wherein the stem length is 19 to 31 nucleotides.

8. A method of suppressing expression of a gene encoding a protein *in vitro,* comprising introducing the single-chain circular RNA according to any one of claims 1 to 3 to human-derived cells, and impairing a target RNA by the single-chain circular RNA to inhibit translation of the target RNA into the protein in a sustained manner.

9. A method of suppressing expression of a gene encoding a protein, comprising introducing *in vitro* the single-chain circular RNA according to any one of claims 1 to 3 to cells of a non-human animal or plant, and impairing a target RNA by the single-chain circular RNA to inhibit translation of the target RNA into the protein in a sustained manner.

10. A pharmaceutical composition comprising the single-chain circular RNA according to any one of claims 1 to 3 as an active ingredient.

## Patentansprüche

1. Ringförmige Einzelketten-RNA, die eine anhaltend freigesetzte oder langsam freigesetzte RNA-Interferenz-Wirkung hat, **dadurch gekennzeichnet, dass** die ringförmige Einzelketten-RNA eine Sense-Strang-Sequenz, eine Antisense-Strang-Sequenz, die zu der Sense-Strang-Sequenz komplementär ist, zwei identische oder unterschiedliche Loop-Sequenzen zwischen dem Sense-Strang und dem Antisense-Strang, die beide Stränge verbindet, umfasst, wobei der Sense-Strang und der Antisense-Strang unter Bildung eines Stems gepaart sind, und wobei jede der zwei Loop-Sequenzen eine Länge von 6 bis 9 Nukleotiden hat.

2. Ringförmige Einzelketten-RNA gemäß Anspruch 1, wobei die Expression einer Target-RNA 0,4 oder weniger 24 Stunden nach Einführung der ringförmigen Einzelketten-RNA in eukaryotische Zellen im Vergleich zu Kontrollzellen, deren Expressionslevel der Target-RNA auf 1 eingestellt ist, ist.

3. Ringförmige Einzelketten-RNA gemäß Anspruch 1 oder 2, wobei 70% oder mehr davon nach 8 Stunden in humanem Serum aufrechterhalten ist.

4. Verfahren zur Herstellung einer ringförmigen Einzelketten-RNA gemäß einem der Ansprüche 1 bis 3, umfassend Synthetisieren eines Sense-Stranges und eines Antisense-Stranges, die beide eine Nukleotidsequenz mit ungepaarten Nukleotiden am 5'-Ende und 3'-Ende umfassen, und gleichzeitiges Ligieren des Nukleotids am 5'-Ende der Nukleotidsequenz mit ungepaarten Nukleotiden im Sense-Strang mit dem Nukleotid am 3'-Ende der Nukleotidsequenz mit ungepaarten Nukleotiden im Antisense-Strang und vice versa unter Verwendung einer Ligase,
wobei die Nukleotidsequenz mit ungepaarten Nukleotiden am 5'-Ende des Sense-Stranges und die Nukleotidsequenz mit ungepaarten Nukleotiden am 3'-Ende des Antisense-Stranges unter Bildung eines Loops aneinander gebunden werden, die Nukleotidsequenz mit ungepaarten Nukleotiden am 3'-Ende des Sense-Stranges und die Nukleotidsequenz mit ungepaarten Nukleotiden am 5'-Ende des Antisense-Stranges unter Bildung eines Loop aneinander gebunden werden und der Sense-Strang und der Antisense-Strang unter Bildung eines Stem gepaart werden.

5. Verfahren gemäß Anspruch 4, das außerdem Phosphorylierung jedes 5'-Endes der Nukleotidsequenz mit ungepaarten Nukleotiden im Sense-Strang und der Nukleotidsequenz mit ungepaarten Nukleotiden im Antisense-Strang umfasst.

6. Verfahren gemäß Anspruch 4 oder 5, wobei die Loop-Sequenzen zueinander identisch oder voneinander verschieden sind.

7. Verfahren gemäß einem der Ansprüche 4 bis 6, wobei die Stem-Länge 19 bis 31 Nukleotide ist.

8. Verfahren zur Unterdrückung der Expression eines Gens, das ein Protein codiert, *in vitro,* umfassend Einführen der ringförmigen Einzelketten-RNA gemäß einem der Ansprüche 1 bis 3 in vom Menschen stammende Zellen und Beeinträchtigen einer Target-RNA durch die ringförmige Einzelketten-RNA unter Inhibierung der Translation der Target-RNA in das Protein in anhaltender Art.

9. Verfahren zum Supprimieren der Expression eines Gens, das ein Protein codiert, umfassend Einführen *in vitro* der ringförmigen Einzelketten-RNA gemäß einem der Ansprüche 1 bis 3 in Zellen eines nichtmenschlichen Tiers oder einer Pflanze und Beeinträchtigen einer Target-RNA durch die ringförmige Einzelketten-RNA unter Inhibierung der Translation der Target-RNA in das Protein in anhaltender Art.

10. Pharmazeutische Zusammensetzung, die die ringförmige Einzelketten-RNA gemäß einem der Ansprüche 1 bis 3 als aktives Ingrediens umfasst.

## Revendications

1. ARN circulaire simple-chaîne à effet persistant ou ralenti d'interférence par ARN, **caractérisé en ce que** cet ARN circulaire simple-chaîne comprend une séquence de brin sens, une séquence de brin anti-sens qui est complémentaire de la séquence de brin sens, et deux séquences de boucles, identiques ou différentes, situées entre le brin sens et le brin anti-sens et raccordant ces deux brins, étant entendu que le brin sens et le brin anti-sens sont appariés et forment une tige, et que chacune des deux séquences de boucles est longue de 6 à 9 nucléotides.

2. ARN circulaire simple-chaîne conforme à la revendication 1, avec lequel le niveau d'expression d'un ARN cible, 24 heures après l'introduction de cet ARN circulaire simple-chaîne dans des cellules eucaryotes, est inférieur ou égal à 0,4, si le niveau d'expression de cet ARN cible dans des cellules témoins est posé égal à 1.

3. ARN circulaire simple-chaîne conforme à la revendication 1 ou 2, dont une fraction d'au moins 70 % se maintient après 8 heures dans du sérum humain.

4. Procédé de préparation d'un ARN circulaire simple-chaîne conforme à l'une des revendications 1 à 3, comportant le fait de synthétiser un brin sens et un brin anti-sens comprenant tous deux des parties de séquence constituées de nucléotides non-appariés, à l'extrémité 5' et à l'extrémité 3', et le fait de ligaturer simultanément, au moyen d'une ligase, le nucléotide placé à l'extrémité 5' de la partie de séquence à nucléotides non-appariés du brin sens avec le nucléotide placé à l'extrémité 3' de la partie de séquence à nucléotides non-appariés du brin anti-sens, et vice versa,
grâce à quoi la partie de séquence à nucléotides non-appariés située à l'extrémité 5' du brin sens et la partie de séquence à nucléotides non-appariés située à l'extrémité 3' du brin anti-sens sont raccordées l'une à l'autre de manière à former une boucle, la partie de séquence à nucléotides non-appariés située à l'extrémité 3' du brin sens et la partie de séquence à nucléotides non-appariés située à l'extrémité 5' du brin anti-sens sont raccordées l'une à l'autre de manière à former une boucle, et le brin sens et le brin anti-sens sont appariés de manière à former une tige.

5. Procédé conforme à la revendication 4, qui comporte en outre le fait de phosphoryler chacune des extrémités 5' de la partie de séquence à nucléotides non-appariés du brin sens et de la partie de séquence à nucléotides non-appariés du brin anti-sens.

6. Procédé conforme à la revendication 4 ou 5, dans lequel les séquences de boucles sont identiques l'une à l'autre ou différentes l'une de l'autre.

7. Procédé conforme à l'une des revendications 4 à 6, dans lequel la tige est longue de 19 à 31 nucléotides.

8. Procédé de suppression *in vitro* de l'expression d'un gène codant une protéine, comportant le fait d'introduire dans des cellules dérivées d'un humain un ARN circulaire simple-chaîne conforme à l'une des revendications 1 à 3, et le fait d'altérer un ARN cible avec cet ARN circulaire simple-chaîne, de manière à inhiber de façon persistante la traduction de l'ARN cible en ladite protéine.

9. Procédé de suppression de l'expression d'un gène codant une protéine, comportant le fait d'introduire *in vitro*, dans des cellules dérivées d'un animal non-humain ou d'un végétal, un ARN circulaire simple-chaîne conforme à l'une des revendications 1 à 3, et le fait d'altérer un ARN cible avec cet ARN circulaire simple-chaîne, de manière à inhiber de façon persistante la traduction de l'ARN cible en ladite protéine.

10. Composition pharmaceutique comprenant, en tant qu'ingrédient actif, un ARN circulaire simple-chaîne conforme à l'une des revendications 1 à 3.


### SEQUENCE LISTING

<110> RIKEN
   Otsuka Pharmaceutical Co., Ltd.
   Hayashi Kasei Co., Ltd.
<120> A single-chain circular RNA and method of producing the same
<130> PH-3542PCT
<150> JP 2007-125045
<150> 2007-05-09
<160> 13
<170> PatentIn version 3.4
<210> 1
   <211> 28
   <212> RNA
   <213> Artificial
<220>
   <223> Synthetic
<400> 1
   gagacuuacg cugaguacuu cgauucaa 28
<210> 2
   <211> 28
   <212> RNA
   <213> Artificial
<220>
   <223> Synthetic
<400> 2
   gagaucgaag uacucagcgu aaguucaa 28
<210> 3
   <211> 56
   <212> RNA
   <213> Artificial
<220>
   <223> Synthetic
<400> 3
   gagacuuacg cugaguacuu cgauucaaga gaucgaagua cucagcguaa guucaa 56
<210> 4
   <211> 21
   <212> RNA
   <213> Artificial
<220>
   <223> Synthetic
<400> 4
   gugcgcugcu ggugccaacu u 21
<210> 5
   <211> 21
   <212> RNA
   <213> Artificial
<220>
   <223> Synthetic
<400> 5
   guuggcacca gcagcgcacu u 21
<210> 6
   <211> 28
   <212> RNA
   <213> Artificial
<220>
   <223> Synthetic
<400> 6 28
   gagagugcgc ugcuggugcc aacuucaa 28
<210> 7
   <211> 28
   <212> RNA
   <213> Artificial
<220>
   <223> Synthetic
<400> 7 28
   gagaguuggc accagcagcg cacuucaa 28
<210> 8
   <211> 32
   <212> RNA
   <213> Artificial
<220>
   <223> Synthetic
<400> 8 32
   gagaagugcg cugcuggugc caacccuuuc aa 32
<210> 9
   <211> 32
   <212> RNA
   <213> Artificial
<220>
   <223> Synthetic
<400> 9
   gagaagggug ggcaccagca gcgcacuuuc aa 32
<210> 10
   <211> 36
   <212> RNA
   <213> Artificial
<220>
   <223> Synthetic
<400> 10
   gagaaaagug cgcugcuggu gccaacccua uuucaa 36
<210> 11
   <211> 36
   <212> RNA
   <213> Artificial
<220>
   <223> Synthetic
<400> 11
   gagaauaggg uuggcaccag cagcgcacuu uuucaa 36
<210> 12
   <211> 40
   <212> RNA
   <213> Artificial
<220>
   <223> Synthetic
<400> 12
   gagaucaaag ugcgcugcug gugccaaccc uauucuucaa 40
<210> 13
   <211> 40
   <212> RNA
   <213> Artificial
<220>
   <223> Synthetic
<400> 13
   gagagaauag gguuggcacc agcagcgcac uuugauucaa 40